(19) Europäisches Patentamt
European Patent Office
Office européen des brevets

(11) EP 4 763 124 A1

(12) **EUROPEAN PATENT APPLICATION**

(43) Date of publication:
24.06.2026 Bulletin 2026/26

(21) Application number: 24222680.1

(22) Date of filing: 21.12.2024

(51) International Patent Classification (IPC):
*A61B 34/30* (2016.01)

(52) Cooperative Patent Classification (CPC):
A61B 34/30

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Microsure B.V.
5692 EA Son (NL)

(72) Inventors:
- CHATROU, Martijn Lambertus Laurentius
5692 VN Son en Breugel (NL)
- VAN LIESHOUT, Richard
6603 KT Wijchen (NL)
- VAN GERVEN, Lars
5382 KC Vinkel (NL)
- BOSSCHER, Kasper
5616 GX Eindhoven (NL)

(74) Representative: DTS Patent- und Rechtsanwälte
PartmbB
Brienner Straße 1
80333 München (DE)

(54) **ROBOT ARM FOR USE IN SURGERY, MICROSURGERY OR SUPER-MICROSURGERY**

(57) The present invention provides a robot arm (100) microsurgery or super-microsurgery, said robot arm (100) comprising at least one arm element (102, 116, 124) and at least one direct drive (134, 136), wherein the at least one direct drive is configured and arranged to actuate the at least one arm element (102, 116, 124). The invention further provides a surgical, microsurgical or super-microsurgical robotic system (200) comprising the robot arm described above.

Fig. 3

**Description**

The present invention belongs to the technical field of robots for use in surgical, microsurgical or super-microsurgical procedures.

In particular, the present invention relates to a robot arm, in particular a Patient Side Unit (PSU) arm, for use in surgery, microsurgery or super-microsurgery, and a robotic system including the same.

For instance, the robot arm can be used to perform anastomoses.

Microsurgery and super-microsurgery currently play a significant role in medical practice.

In surgical procedures, in particular when microsurgery or super-microsurgery are concerned, robots are used for precise surgical handlings on the submillimeter scale.

The use of robots facilitates accurate movements with micrometer precision without substantial tremor, thereby superseding the limitations of human hand manipulation.

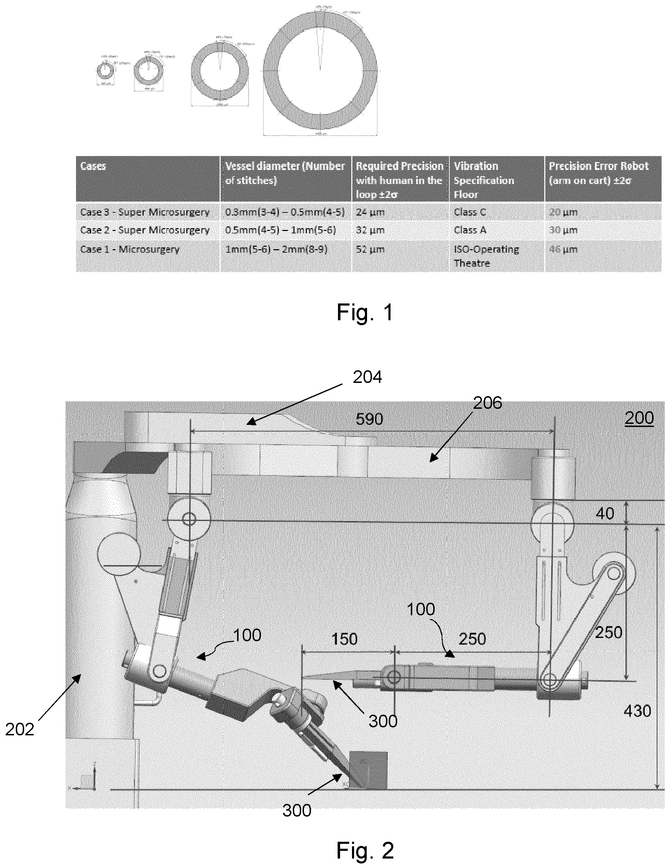

The required accuracy for a surgical robotic system in three different cases regarding microsurgical/super-microsurgical operations is shown in **Fig. 1.**

Here, three to six stitches are usually required for each vessel.

Robotic systems shall provide a sufficient dexterity, enabling the operator (e.g. a surgeon) to perform complex movements in the microsurgical or super-microsurgical workspace, and also avoid the risk of collision with other objects (or even the patient) during manipulation.

Also, it is important that parts of the robotic system, in particular the robot arm, do not interfere with and thus obstruct the microscopic view of the operator.

The latter aspect is particularly relevant considering the environmental volume constraints of surgical, in particular microsurgical and super-microsurgical workspaces.

Robots for use in surgical, microsurgical or super-microsurgical procedures are known in the art.

In particular, one known approach involves the use of laparoscopic systems.

Here, surgical instruments are attached to relatively long rods.

However, the use of long rods renders the system susceptible to disturbances at the tip, which may hinder precision and maneuverability.

Another known approach involves the use of robotic systems having six degrees of freedom using indirect drive mechanisms, such as gears or the like.

However, the use of indirect drive mechanisms may introduce imprecision, friction, backlash, and compliance-related issues, especially when dealing with extremely fine and delicate surgical tasks.

EP2731535 discloses a microsurgical robotic device for providing robotic assistance during tasks that require long-term user concentration and high precision. One or multiple master-slave units are coupled to a central microscope-based suspension structure. The microsurgical robotic devices pay attention to motion scaling and tremor filtration in a 6 Degrees-of-Freedom master-slave setup with force feedback. An extra degree of freedom is included to actuate a 1-DOF instrument tip.

A drawback of this known robotic device is that link lengths are suboptimal.

As a consequence, it is not possible to provide the operator with sufficient dexterity that is required to perform a wide range of microsurgical or super-microsurgical techniques (e.g., anastomoses).

A further drawback is that parts of the robot, in particular the robot arm, may interfere with and thus obstruct the operator's view on the surgical site during surgery.

An improved solution is provided in EP22169326, filed in the name of the same applicant, disclosing a robot arm for use in surgery, microsurgery or super-microsurgery comprising a first, proximal arm element having a first arm length, a second, intermediate arm element having a second arm length, and a third, distal arm element having a third arm length, where enhanced precision and dexterity are obtained through the definition of an optimized ratio between arm lengths.

In particular, the ratio between the first to third arm element lengths is defined as follows:

$$(1.00 +- 10\%) : \{ (1.00 \text{ to } 1.05) +- 10\% \} : (0.60 +- 10\%).$$

In the light of the increasing relevance of microsurgery and super-microsurgery in medical practice, there is the need for a solution allowing performing intricate surgical procedures with an even higher level of dexterity and precision, thereby enhancing the overall quality of microsurgical and super-microsurgical procedures and patient outcomes.

In view of the above, it is an object of the present invention to provide an improvement for a robot arm for use in surgery, microsurgery or super-microsurgery, inter alia in that the robot arm is capable of actuating at least one arm element and/or at least one degree of freedom with minimal mechanical compliance, thereby preserving precise control down to the micrometer level

It is also an object of the present invention to provide a robot arm as defined above, said robot arm being able to improve the overall safety conditions while performing surgical, microsurgical or super-microsurgical procedures, in particular by reducing the risk of accidental damage to delicate tissues of the patient.

The above objects are obtained by the provision of a robot arm according to claim 1.

In particular, a robot arm for use in surgery, microsurgery or super-microsurgery is provided, said robot arm comprising:

at least one arm element, and

at least one direct drive,

wherein the at least one direct drive is configured and arranged to actuate the at least one arm element.

The present invention provides a robot arm, in particular a Patient Side Unit (PSU) arm.

The robot arm is designed for use in for use in surgical, preferably microsurgical or super-microsurgical procedures, where the highest level of precision is required.

The robot arm comprises at least one arm element.

The robot arm further comprises at least one direct drive.

The at least one direct drive is direct drive is configured and arranged to actuate the at least one arm element.

The invention is based on the basic idea that, by directly actuating the at least one arm element through a respective direct drive, e.g. a frameless direct drive motor, said at least one arm element can be actuated with minimal mechanical compliance, thereby preserving highly-precise control down to the micrometer level. In particular, this approach allows overcoming mechanical complexity that characterizes indirect-drive systems, enhancing precision and reducing the occurrence of unwanted hysteresis and backlash.

Accordingly, even intricate microsurgical or super-microsurgical procedures can be performed with high level of precision.

Direct actuation allows significantly reducing mechanical complexity, thereby improving positioning controllability of the surgical instrument.

This also provides the operator with the feeling of directly manipulating the surgical instrument with his/her hand, because the instrument closely follows the operator's desired movements, remaining insensitive to disruptions. Otherwise stated, the robot arm behaves like an extension of the operator's hand, allowing manipulating the surgical instrument with enhanced precision and ease.

Further, the configuration of the robot arm can be better tailored to the operator, in particular by means of control/filter settings of the robot joints. This further allows preventing the occurrence of delays in responding to the operator's movement, and optimizing steepness, and damping of the response In particular, the invention allows obtaining the following advantages compared to the solutions according to the state of the art:

- reduction of play, friction, wear that characterize known robot arms relying on the use of gearboxes, which may be prejudicial to the overall accuracy and ease of control;

- improved level of control for the surgeon. Known solutions involving large transmission and gearboxes are substantially insensitive to load variations and tip disturbances, but have the drawback that they can be controlled only to a limited extent. The robot arm of the invention, although more sensitive, can however be controlled with a high level of accuracy. Overall, disturbances at the tip can be deemed negligible, since these forces are significantly small and thus hardly felt by the operator during manipulation;

- improved dynamics and positioning accuracy;

- increased sensitivity to deviations in position/force, which can be immediately detected (e.g. through control means such as a controller), so that necessary actions can be taken without delay;

- improved maneuverability, giving the operator the feeling of directly manipulating the surgical instrument with his/her hand. Also, the robot arm is highly adjustable based on the needs and/or preferences of the operator.

The robot arm may include a first, proximal arm element.

The robot arm may also include a second, intermediate arm element.

The proximal arm element is connected to a first, proximal joint and a second, intermediate joint.

In particular, the proximal joint comprises a first proximal joint element configured to perform a first roll movement and a second proximal joint element configured to perform a first pitch movement.

In particular, the intermediate joint comprises a first intermediate joint element configured to perform a second roll movement and a second intermediate joint element configured to perform a second pitch movement.

The intermediate arm element is connected to the intermediate joint and a third, distal joint.

In particular, the distal joint comprises a first distal joint element configured to perform a third roll movement and a second distal joint element configured to perform a third pitch movement.

There may be also a third, distal arm element provided in the robot arm.

The distal arm element is connected to the distal joint.

The robot arm may be configured to have six degrees of freedom at its tip.

In particular, the robot arm may configured such that at least one degree of freedom is directly actuated by a respective direct drive.

By means of direct actuation, one or more degrees of freedom, e.g., five of the six degrees of freedom, can be actuated with minimal mechanical compliance, thereby allowing performing surgical, microsurgical or super-microsurgical procedures with the highest level of precision at the instrument tip.

Each of the first proximal joint element, the second proximal joint element, the first intermediate joint element, the second intermediate joint element, and the second distal joint element may comprise a direct drive, e.g. a frameless direct drive motor, and a motor brake.

In the second distal joint element, driving is carried out through a belt.

This allows reducing mass and volumes toward the end effector (and, eventually, the patient), in turn resulting in a reduction of required motor torques.

This has the further advantage that clearance around the patient and the overall visibility conditions for the surgeon can be improved. Also, the risk of accidental collisions with the patient can be prevented.

Space constraints that characterize the first distal joint element do not allow for the provision of a direct drive as in the case of the other joint elements of the robot arm.

Therefore, the first distal joint element may be provided with a servo motor with integrated backlash-free gearbox.

The first distal joint element is mostly involved in rotating the surgical instrument, which does not directly affect positioning of the instrument tip. Accordingly, even in case an indirect drive mechanism implemented through a servo motor is used, this does not hinder the overall level of precision in manipulating the instrument.

The use of a compact servo motor, low in mass and volume, is also preferable considering the space constraints in the first distal joint element.

The robot arm may further comprise a mass compensation means.

This allows improving the overall stability and precision of the robot arm during use.

Conveniently, the mass compensation means may be arranged in the second proximal joint element.

Also, optionally in addition to the above, the mass compensation means may be arranged in the second intermediate joint element.

For example, the mass compensation means may be arranged in the second proximal joint element and comprise a first spring system acting in a first direction and a second spring system acting in a second direction, said second direction being opposite to said first direction.

The positioning and stiffness of the first and second spring systems are defined in such way that the mass of the intermediate joint and the distal joint are optimally compensated at respective nominal operation positions.

Preferably, the first spring system comprises a double spring, while the second spring system comprises a single spring.

The pre-load of the springs in each spring system can be adjusted by using adjustment screws.

The use of a double spring for the first spring system allows obtaining a reduced thickness. In fact, a single spring would require a larger diameter, this resulting in an increased volume.

As a further example, optionally in addition to the above, the mass compensation means may be arranged in the second intermediate joint element and comprises a third spring system.

In particular, the third spring system comprises a single spring.

Advantageously, the third spring system may be arranged next to a pulley system of the second intermediate joint element, this resulting in a reduced volume.

Preferably, the mass compensation means is arranged both in the second proximal joint element and the second intermediate joint element.

The provision of mass compensation means in the second proximal joint element and/or the second intermediate joint element allows decreasing required motor torque thereby decreasing heat dissipation, resulting in improvements in energy efficiency.

Advantageously, the six degrees of freedom of the robot arm may comprise three translational degrees of freedom and three rotational degrees of freedom.

Accordingly, it is possible to perform surgical, in particular microsurgical and super-microsurgical operations (e.g., anastomoses) with greater accuracy and enhanced maneuverability.

Advantageously, when first to third arm elements are provided in the robot arm, the ratio between the first to third arm element lengths can be:

$$(1.00 +- 10\%) : \{ (1.00 \text{ to } 1.05) +- 10\%\} : (0.60 +- 10\%).$$

Preferably, the ratio between the first to third arm element lengths can be:

$$(1.00 +- 3\% \text{ to } +- 5\%) : \{(1.00 \text{ to } 1.05) +- 3 \text{ to } +- 5\%\} : (0.60 +- 3\% \text{ to } +- 5\%).$$

The disclosed ratios have been found by experiment and experience and have shown to be very effective to achieve, inter alia, the above-mentioned objects.

Advantageously, the first arm element length is between 225 and 275 mm.

Advantageously, the second arm element length is between 231.75 and 283.25 mm.

Advantageously, the third arm element length is between 135 and 165 mm.

Preferably, the first arm length is 250 mm.

Preferably, the second arm length is 257.5 mm.

Preferably, the third arm element is 150 mm.

Precision and accuracy of movements as well as maneuverability of the robot arm can be further improved by the specific features and ranges of motion of the proximal, intermediate, and distal joints.

In particular, the proximal joint may be characterized by the following ranges of motion:

first roll: -41° to +41° with respect to the first roll rotation axis, and/or

first pitch: -23° to +44° with respect to the first pitch rotation axis.

In particular, optionally in addition to the above, the intermediate joint may be characterized by the following ranges of motion:

second roll: -180° to 180° with respect to the second roll rotation axis, and/or

second pitch: -8° to +90° with respect to the second pitch rotation axis.

In particular, optionally in addition to the above, the distal joint may be characterized by the following ranges of motion:

third roll: +-inf, and/or

third pitch: -185° to 185° with respect to the third pitch rotation axis.

The robot arm may further comprise a button for simultaneous disengagement of the motor brakes.

Accordingly, the operator is enabled to easily and promptly trigger simultaneous disengagement of all motor brakes.

Conveniently, the button may be placed at the tip of the robot arm.

Accordingly, the button can be more easily operated by the operator.

Preferably, the button is placed on top of the first distal joint element of the robot arm.

This specific location for the button was chosen by the inventors after performing tests with a full-scale mock-up.

The first distal joint element may comprise an end-effector connection means.

In particular, said end-effector connection means comprises electrical and mechanical interfaces for connection to an end-effector of the robot arm.

Due to the space constraints in both the end-effector and end-effector connection means, it is preferable to avoid the use of cables.

Conveniently, the end-effector may be connected to the first distal joint element through a spring-loaded connection means.

Each of the first proximal joint element, the second proximal joint element, the first intermediate joint element, the second intermediate joint element, and the second distal joint element may include a motor stack.

In particular, the motor stack comprises a housing that encloses the direct drive and the motor brake.

Advantageously, the motor stack may further comprise an encoder, arranged within the housing.

Advantageously, optionally in addition to the above, the motor stack may further comprise a drive PCB, arranged within the housing.

Advantageously, optionally in addition to the above, the motor stack may further comprise one or more bearings, arranged within the housing.

For instance, the motor stack may include first and second bearings.

Preferably, in each bearing, the rotor is pre-loaded with respect to the stator, so that no clearance is generated between balls and the rotor/stator.

Accordingly, the occurrence of backlash can be largely prevented.

In the second distal joint element, driving is carried out through a belt. Each of the first intermediate joint element, the second intermediate joint element, the first distal joint element, and the second distal joint element may comprise a respective cover.

In particular, the covers can be made of an electrically insulating material.

This provides further protection.

In particular, optionally in addition to the above, the covers can be made of a thermally insulating material.

Accordingly, it is easier to comply with temperature requirements during surgery.

Preferably, the covers are made of a material that is both electrically and thermally insulating.

The cover of the first intermediate joint element and the cover of the second distal joint element can be removable.

This facilitates cleaning operations.

The remaining covers are not removable.

In order to reduce temperature build-up, some components of the robot arm may be configured to receive a reduced voltage.

In particular, the first intermediate joint element and the second distal joint element may be configured to receive a reduced voltage of 24V.

In particular, optionally in addition to the above, the first distal joint element may be configured to receive an even more reduced voltage of 12V.

Lowering the applied voltage in some parts of the robot arm allows improving the overall safety conditions.

Accordingly, patient protection measures can be mitigated.

Additionally, the efficiency in the electronics of the robot arm can be improved because there is a smaller difference between the applied and the supplied voltage.

A voltage of 48V is applied to the remaining parts of the robot arm.

Optionally, a sterile drape can be used to cover the robot arm for maintaining sterility conditions in the course of the surgical, microsurgical or super-microsurgical procedures.

The present invention further provides a surgical, microsurgical or super-microsurgical robot system including, inter alia, the robot arm described above.

In particular, the system may be configured and adapted to perform anastomoses.

Th system comprises a base station.

The system further comprises a base column.

There is also a suspension arm provided, in particular carried by the base column.

The suspension arm is able to rotate about the longitudinal axis of the base column.

Still further, the system includes a fork element, carried by the suspension arm.

The at least one robot arm, such as the robot arm described above, is carried by said fork element.

The at least one robot arm is configured to carry a respective surgical instrument, mounted at its distal end.

Further details and advantages of the present invention shall now be disclosed in connection with the drawings, where:

Fig. 1     is a diagram showing the required accuracy for a robotic system in three different cases concerning microsurgery or super-microsurgery;

Fig. 2     is a partial, perspective view of a surgical robotic system including two robot arms, according to an embodiment of the present invention;

Fig. 3     is a perspective view of a robot arm for use in surgery, microsurgery or super-microsurgery procedures, according to an embodiment of the present invention;

Fig. 4     is a view similar to that of **Fig. 3**, but with removed parts;

Fig. 5     is a perspective view similar to **Fig. 3** but from a different perspective and further showing the six degrees of freedom of the robot arm;

Fig. 6     is a schematic view in cross section of a motor stack for use in the robot arm of **Fig. 3**, in particular for the first proximal joint element, the second proximal joint element, the first intermediate joint element, the second intermediate joint element and the second distal joint element. The motor stack encloses, inter alia, the direct drive and the moto brake;

**Fig. 7**      is a perspective, detail view of the first proximal joint element of the robot arm of **Fig. 3**;

**Fig. 8**      is a perspective, detail view of the second proximal joint element of the robot arm of **Fig. 3**;

**Fig. 9**      is a perspective, detail view of the first intermediate joint element of the robot arm of **Fig. 3**;

**Fig. 10**     is a perspective, detail view of the second intermediate joint element of the robot arm of **Fig. 3**;

**Fig. 11**     is a schematic view showing the layout of the intermediate joint, with omitted parts;

**Fig. 12**     is a schematic view similar to that of **Fig. 11**, but from the opposite side;

**Fig. 13**     is a schematic view showing, inter alia, a detail of the mass compensation means in the second intermediate joint element of the robot arm of **Fig. 3**;

**Fig. 14**     is a schematic view showing, inter alia, a detail of the mass compensation means in the second proximal joint element of the robot arm of **Fig. 3;**

**Fig. 15**     is diagram showing performance measurements for the first and second proximal joint elements and the first and second intermediate joint elements of the robot arm according to an embodiment of the invention, such as the robot arm of **Fig. 3;**

**Fig. 16**     is a perspective, detail view of the first distal joint element of the robot arm of **Fig. 3,** with omitted parts;

**Fig. 17**     is a view similar to that of **Fig. 13,** but from a different perspective;

**Fig. 18**     is a schematic, detail view showing the layout of the second distal joint element of the robot arm of **Fig. 3;**

**Fig. 19**     is a perspective view of a detail of the covers of the robot arm of **Fig. 3;**

**Fig. 20**     is a schematic view showing a ball bearing system according to the prior art. Here, a clearance is defined between the balls and the rotor/stator;

**Fig. 21**     is a schematic view showing a ball bearing system as used in the robot arm according to the present invention, such as the robot arm shown in **Fig. 3**. Here, no clearance is defined between the balls and the rotor/-stator, this resulting in backlash avoidance;

**Fig. 22**     is a schematic, perspective view showing how ball bearings, in particular a first ball bearing and a second ball bearing, are constrained within the motor stack in an arrangement according to the present invention;

**Fig. 23**     is a perspective; schematic view showing cable routing in the proximal joint of the robot arm of **Fig. 3;**

**Fig. 24**     is a perspective, schematic view showing cable routing in the intermediate joint of the robot arm of **Fig. 3;**

**Fig. 25**     is diagram showing tolerance specifications for encoder placement within the motor stack.

**Fig. 2** shows a partial view of a surgical robotic system 200 for surgical, microsurgical or super-microsurgical operations.

In particular, the robotic system 200 may be adapted to perform anastomoses.

Not shown is that the surgical robotic system 200 includes a base station.

Not shown is that the base station may include a display means for displaying information to one or more operators, and/or provide a user interface for inserting a user input.

Not shown is that the base station may be provided with wheels, allowing for easy placement of the surgical robotic system 200 at a desired location within the operating room.

The surgical robotic system 200 includes a base column 202.

The base column 202 carries a suspension arm 204, which is able to rotate about the longitudinal (perpendicular) axis of the base column 202.

The suspension arm 204 carries a fork element 206, which is able to rotate around a vertical axis mounted on the suspension arm 204.

In the shown configuration, the fork element 206 carries a first robot arm and second robot arm, such as the robot arm 100 described below.

Each of the first and second robot arms carries a respective surgical instrument 300.

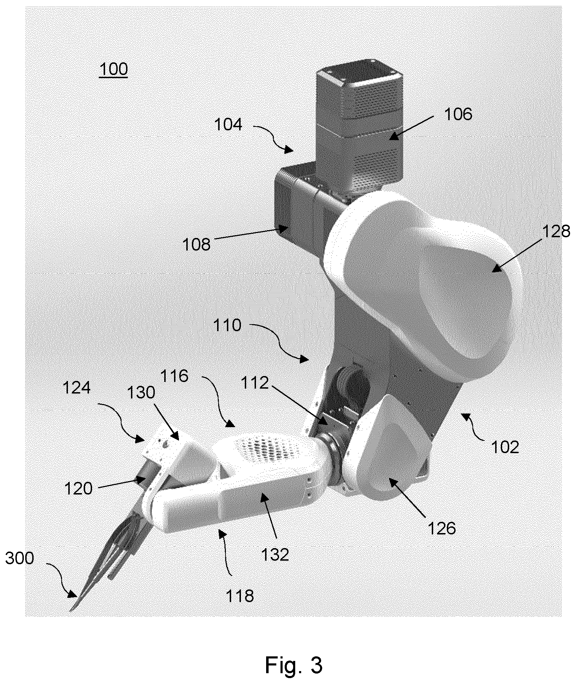

A robot arm 100, in particular a Patient Side Unit (PSU) arm, according to an embodiment of the present invention is shown in **Fig. 3.**

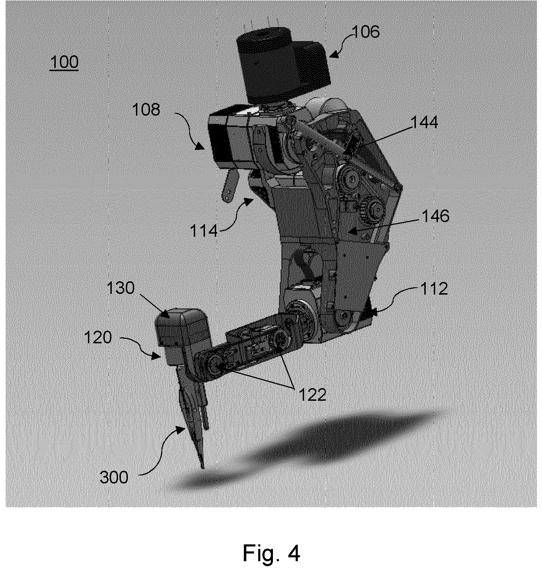

A further view of the robot arm 100, with omitted parts, is provided in **Fig. 4.**

The robot arm 100 is adapted for use in surgical, microsurgical or super-microsurgical procedures.

In particular, the robot arm 100 may be adapted to perform anastomoses.

The robot arm 100 includes at least one arm element. In particular, in the shown embodiment, the robot arm 100 comprises a first, proximal arm element 102, a second, intermediate arm element 116 and a third, distal arm element 124.

Not shown is that different configurations of the robot arm 100 are also possible without prejudice to its functionality.

As a non-limiting example (not shown), the robot arm 100 may only include a first arm element and a second arm element, without the provision of a third arm element,

The proximal arm element 102 is connected to a first, proximal joint 104 and a second, intermediate joint 110.

In particular, the proximal joint 104 comprises a first proximal joint element 106, configured to perform a first roll movement and a second proximal joint element 108, configured to perform a first pitch movement.

In particular, the intermediate joint 110 comprises a first intermediate joint element 112, configured to perform a second roll movement and a second intermediate joint element 114, configured to perform a second pitch movement.

The intermediate element 116 is connected to the intermediate joint 110 and a third, distal joint 118.

In particular, the third, distal joint 118 comprises a first distal joint element 120, configured to perform a third roll movement and a second distal joint element 122, configured to perform a third pitch movement.

The distal arm element 124, connected to the distal joint 118.

The robot arm 100 can be easily draped upon being brought in an appropriate draping position.

The robot arm 100 further incudes at least one direct drive 134.

In particular, said at least one direct drive 134, e.g., a frameless direct drive motor, is configured and arranged to actuate the at least one arm element 102, 116, 124.

For instance, all arm elements 102, 116, 124 of the robot arm 100 can be directly actuated though a respective direct drive 134.

The robot arm 100 is configured to have six degrees of freedom at its tip.

In particular, in the present embodiment, said six degrees of freedom comprise three translational degrees of freedom and three rotational degrees of freedom.

This allows performing anastomoses on microscopic level with enhanced precision.

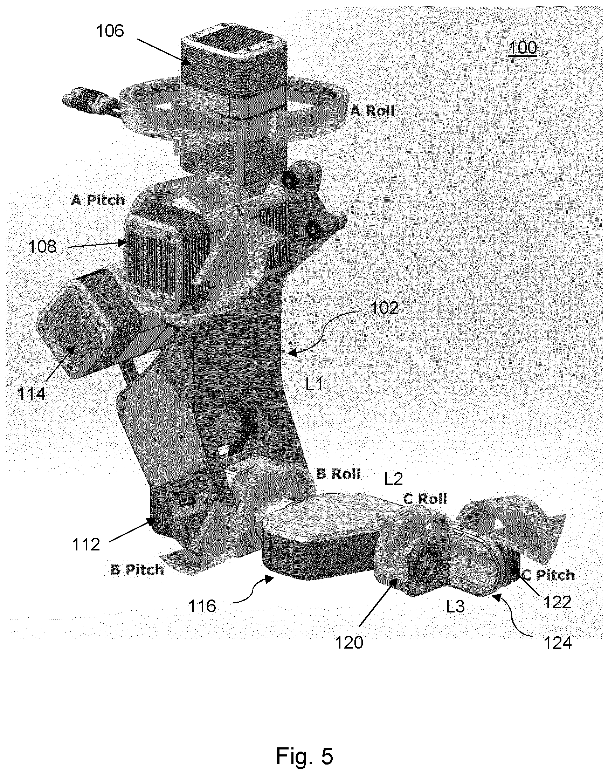

The six degrees of freedom of the robot arm 100 are illustrated in **Fig. 5.**

In the present embodiment, the robot arm 100 is configured such that five among the six degrees of freedom are directly actuated by a respective direct drive 134.

This allows overcoming the mechanical complexity that characterizes indirect drive systems, which may introduce imprecision, friction and other compliance-related issues, especially when dealing with extremely fine and delicate surgical tasks.

Here, each degree of freedom is directly actuated with minimal mechanical compliance, thereby preserving highly precise control down to the micrometer level.

Accordingly, precision can be enhanced and the occurrence of unwanted hysteresis and backlash can be largely prevented.

The remaining degree of freedom is actuated by a servo motor 136.

In the present embodiment, each of the first proximal joint element 106, the second proximal joint element 108, the first intermediate joint element 112, the second intermediate joint element 114, and the second distal joint element 122 comprises a direct drive 134, e.g. a frameless direct drive motor, and a motor brake 138.

Advantageously, backlash-free brakes, such as permanent magnet brakes, can be used.

Further, in order to reduce power consumption by the brakes 138, use can be made of the hysteresis in the engagement and disengagement of each brake 138.

In the present embodiment, each of the first proximal joint element 106, the second proximal joint element 108, the first intermediate joint element 112, the second intermediate joint element 114, and the second distal joint element 122 comprises a motor stack 152 having a housing 154.

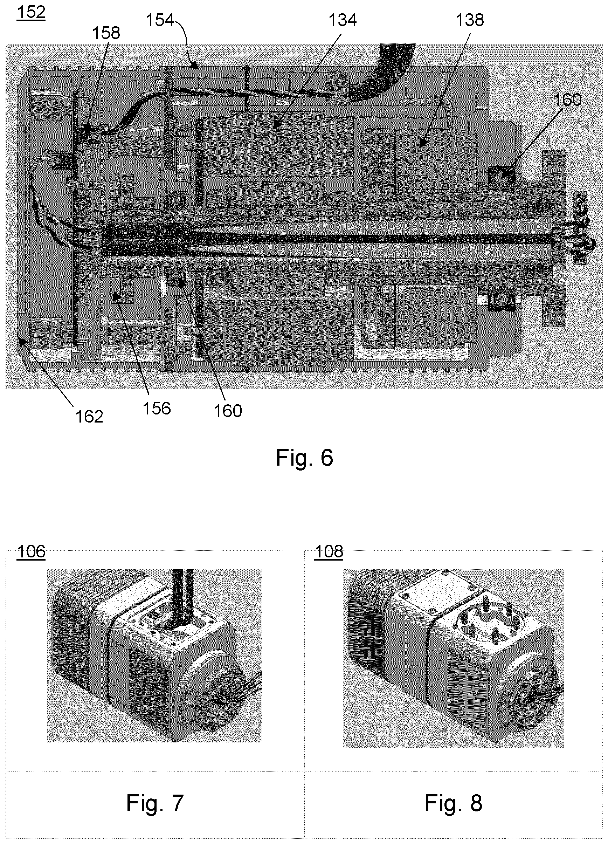

The direct drive 134 and the motor brake 138 of each joint element 106, 108, 112, 114, 122 are arranged within the housing 154, as shown in **Fig. 6**.

Perspective views of the first proximal joint element 106, the second proximal joint element 108, the first intermediate joint element 112 and the second intermediate joint element 114 are provided in **Figs. 7-10,** respectively.

As illustrated in **Fig. 6**, further components can be arranged within the housing 154 of the motor stack 152.

For instance, an encoder 156, a drive PCB 158, and/or one or more ball bearings 160 (first and second ball bearings 160 in the shown configuration) can be provided within the housing 154 (**Fig. 6**).

In order to dissipate the heat generated by the drive PCB 158, said drive PCB 158 can be supported within a heat-conductive foam.

In particular, the heat-conductive foam is adapted to conduct the generated heat to an aluminum panel, which in turn conducts to a cover (which will be described in greater detail in the following).

In the present embodiment, the cover is equipped with a heatsink 162 to radiate heat from the drive PCB 158 to the outer environment (**Fig. 6**).

Accordingly, heat distribution in the motor stack 152 can be improved and the occurrence of hotspots within the motor stack 152 can be reduced.

A specific motor selection as well as an individual design for the motor stack 152 can be provided for each joint 104, 110.

Space constraints characterizing the first distal joint element 120 do not allow for the arrangement of a direct drive, e.g. a frameless direct drive motor, as in the case of joint elements 106, 108, 112, 114, 122.

Therefore, the first distal joint element 120 is provided with a servo motor 136 with integrated backlash-free gearbox 140.

The servo motor 136 operates one of the six degrees of freedom of the robot arm 100.

In particular, it is preferable to use a small-sized servo motor, very low in mass and volume.

In the present embodiment, the robot arm 100 further comprises a control means (not shown) configured to control actuation of the at least one direct drive 134 and/or the servo motor 136, based on preset control parameters.

In the present embodiment, the control means is configured to control actuation of both the at least one direct drive and the servo motor 136.

Advantageously, said control parameters can be fine-tuned by an operator (e.g. a surgeon), customizing responsiveness of the robot arm 100 based on specific requirements of a performed surgical, microsurgical or super-microsurgical procedure.

For example, said control parameters can be set to achieve either an extremely responsive motion or a less responsive motion, based on the current needs.

The robot arm 100 further comprises at least one sensor (not shown), configured to sense disturbances at the tip of a surgical instrument 300 that is connected to the robot arm 100 at its distal end.

The at least one sensor is further configured to provide a feedback signal on the sensed disturbances to said control means.

The control means are configured to perform real-time adaption of said control parameters based on a received feedback from the at least one sensor.

Accordingly, real-time compensation for environmental factors can be achieved, ensuring optimal surgical outcomes.

In particular, by combining real-time adaption of the control parameters based on sensed disturbances at the surgical instrument tip with direct actuation of at least one degree of freedom (here, five of the six degrees of freedom), it is possible to further improve the overall level of precision and adaptability.

Also, the ability to sense and react to disturbances at the tip of the surgical instrument allows enhancing safety conditions, in particular as it reduces the risk of accidental damage to delicate tissues of the patient.

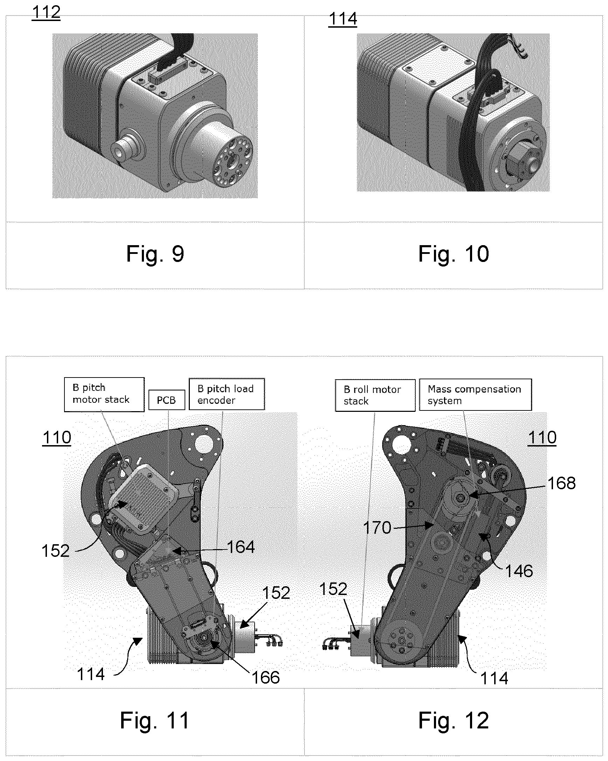

**Figs. 11-12** show different views of the intermediate joint 110 of the robot arm 100 according to the present embodiment.

The intermediate joint 110 comprises the first and second intermediate joint elements 112, 114 described above.

The intermediate joint 110 further comprises an intermediate joint PCB 164 and a load encoder 166 for the second intermediate joint element 114.

There is also a mass compensation means 146 provided, which will be described in greater detail in the following.

The robot arm 100 comprises a mass compensation means 142, 144, 146.

In the present embodiment, the mass compensation means 142, 144, 146 is arranged in the second proximal joint element 108 and the second intermediate joint element 114 (**Figs. 12-14**).

In the second proximal joint element 108, the mass compensation means 142, 144 comprises a first spring system 142, acting in a first direction, and a second spring system 144, acting in a second direction, said second direction being opposite to the first direction.

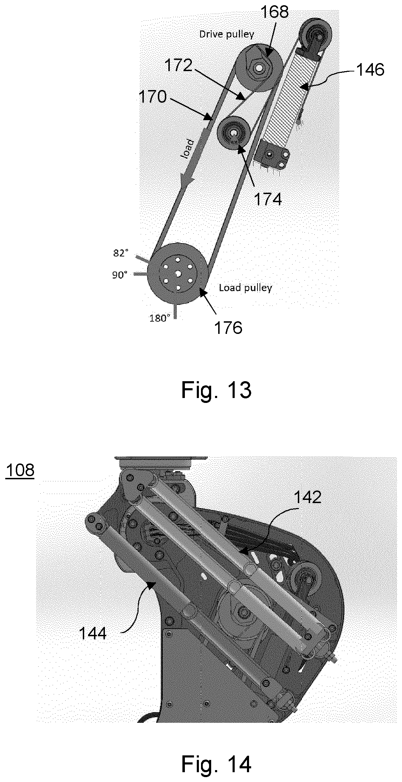

In the present embodiment, the first spring system 142 comprises a double spring, while the second spring system 144 comprises a single spring (**Fig. 14**).

The stiffness and position of the first and second spring systems 142, 144 is defined such that the mass of the intermediate joint 110 and the distal joint 118 are optimally compensated at nominal operation positions.

Due to expected spring deviations in spring constant (+-6%), the pre-load on the springs of the first and second spring systems 142, 144 can be adjusted, e.g. by means of adjustment screws.

The use of a double spring for the first spring system 142 allows reducing thickness.

In fact, a single spring would require a larger diameter, this resulting in an increased volume.

The mass compensation means 146 in the second intermediate joint element 114 comprises a third spring system 146 comprising a single spring (**Figs. 12-13**).

As illustrated in detail in **Fig. 13,** the third spring system 146 is arranged next to a drive pulley 168 and a transmission belt 170, this resulting in a reduced volume.

The load is connected using a steel wire 172 directed over an additional pulley 174 (**Fig. 13**).

In particular, the additional pulley 174 is arranged so that the load on the spring system 146 acts through its axis.

In robot arms, such as the robot arm 100 of the invention, dynamic behavior and stiffness are of significant importance.

Using dynamic FEM analysis, several points of interest have been discovered by the inventors as sources of low stiffness.

Accordingly, a pulley system as shown in **Fig. 13** has been provided in the second intermediate joint element 114, said system being defined by the drive pulley 168, the transmission belt 170 and a load pulley 176.

The transmission belt 170 is preferably made of carbon material and has a width of 12 mm.

The load pulley 176 has a pulley size between 25 to 32 teeth, resulting in a transmission ratio of 1 to 1.28.

In particular, this allows increasing rotational stiffness.

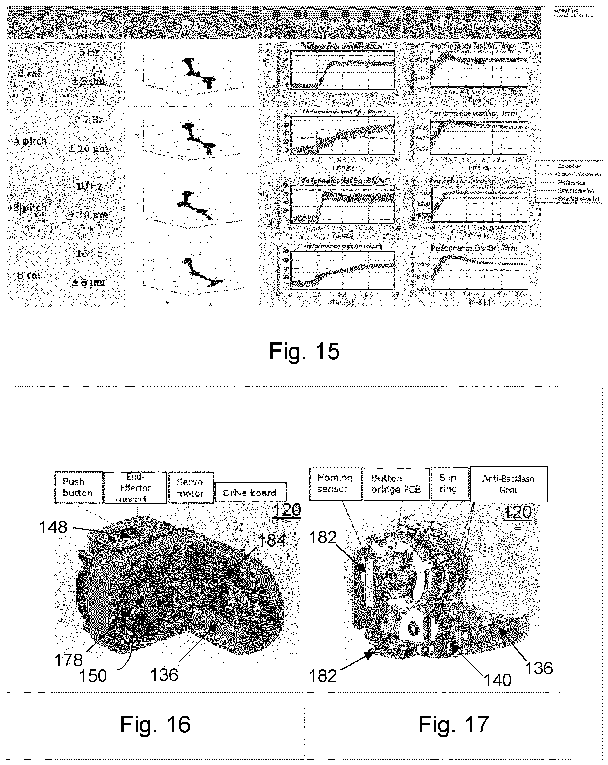

Performance measurements for the first proximal joint element 106, the second proximal joint element 108, the first intermediate joint element 112 and the second intermediate joint element 114 are illustrated in **Fig. 15**.

**Figs. 16-17** show different perspective views of the first distal joint element 120.

As mentioned, space constraint in the first distal joint element 120 do not allow for the provision of a direct drive, e.g. a frameless direct drive motor, as in the other joint elements 106, 108, 112, 114, 112 of the robot arm 100.

Here, a servo motor 136 is provided with integrated backlash-free gearbox 140 (**Fig. 16**).

In particular, transmission from the servo motor 136 to the outgoing axis is implemented according to a two-stage transmission, using a backlash eliminating gear at each stage (**Fig. 17**).

Backlash is further reduced/eliminated through the outgoing axis both in the axial and radial direction by using a spring system.

The servo motor 136 is further provided with an integrated encoder 178.

A backlash-free transmission is defined between the servo motor 136 and an outgoing mechanical interface.

There is a gear ratio between the encoder 178 and the load, and the load side can rotate endlessly.

This results in multiple possible positions at start-up.

Accordingly, a multiturn absolute encoder or a homing sensor 180 on the load side are required.

In the shown configuration, a homing sensor 180 is provided for this purpose (**Fig. 17**).

The first distal joint element 120 further comprises an end-effector connection means 150, as schematically illustrated in **Fig. 16**.

In particular, the end-effector connection means 150 comprises electrical and mechanical interfaces for connection to an end-effector of the robot arm 100.

Due to space constraints both in in the connection means 150 and the end-effector, the use of cables is not convenient.

Advantageously, in the present embodiment, the end-effector is directly connected to the first distal joint element 120 through an electrical spring-loaded connection means 150 (Fig. 16).

The robot arm 100 further comprises a button 148 for simultaneous disengagement of the motor brakes 138.

In the present embodiment, the button 148 is conveniently placed on top of the first distal joint element 120 (and thus close to the end-effector), as shown in **Fig. 16**.

This allows the operator to easily and promptly trigger simultaneous disengagement of all motor brakes 138.

In particular, the button 148 can be part of a cover, which will be described in greater detail in the following.

This specific location for the button 148 was chosen by the inventors after performing tests with a full-scale mock-up.

In the present embodiment, a button bridge PCB 182 can be provided, preferably on a cover of the first distal joint element 120, said button bridge PCB 182 being connected to the button 148 and having an interface toward a drive board 184 of the first distal joint element 120.

In the first distal joint element 120, cable routing to an outgoing interface is implemented by using a slip ring.

This allows the outgoing interface to rotate infinite turns.

Also, PCB-to-PCB connections are used to connect the button bridge PCB 182 to the drive board 184.

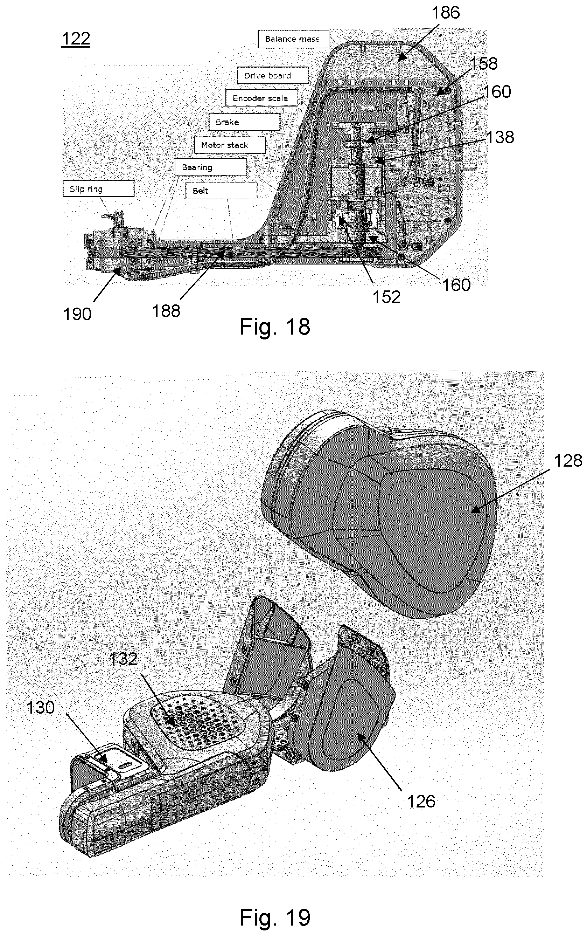

**Figs. 18** schematically illustrates the layout of the second distal joint element 122 of the robot arm 100 according to the present embodiment.

The second distal joint element 122 comprises a motor stack enclosing the direct drive 134 and the motor brake 138.

The motor stack 152 is structurally similar to the motor stack 152 described above, however slightly less complicated.

In particular, since the cables are running through the joint element 122 (and not through an inner shaft), the motor stack 152 does not require any internal pre-loading, this resulting in a simpler design.

In particular, for optimal mass placement for the axes of the second proximal joint element 108 and the second intermediate joint element 114, the direct drive 134 is arranged toward the axis of the second distal joint element 122 (**Fig. 18**).

In the present embodiment, a balance mass 186 is provided in the upper portion of the second distal joint element 122, in particular to balance out the gravitational load for the first distal joint element 120.

In the second distal joint element 122, driving is carried out through a belt 188 (**Fig. 18**).

In particular, the belt 188 is pre-loaded, such that the bearings 160 are also pre-loaded, thereby preventing the occurrence of radial backlash.

On the other hand, axial backlash is removed by using a spring system providing pre-load in the axial direction.

Backlash occurring in the motor stack 152 has no influence at the instrument tip and is therefore considered negligible.

As mentioned, cables are directed though the second distal joint element 122, as illustrated in **Fig. 18**.

A slip ring 190, e.g. including eight pins, can be used to prevent cable torsion.

This is particularly useful considering that the second distal joint element 122 has a quire large range of movement.

Generally, the applied voltage over the elements of the robot arm 100 is 48V.

Nevertheless, in order to reduce temperature build-up, the voltage is reduced in some of the elements of the robot arm 100.

In particular, according to the present embodiment, the first intermediate joint element 112 and the second distal joint element 122 are configured to receive a voltage of 24V.

Further, the first distal joint element 120 is configured to receive an even more reduced voltage of 12V.

Lowering the applied voltages increases the overall safety conditions of the robot arm 100, thereby improving patient protection.

Each of the first intermediate joint element 112, the second intermediate joint element 114, the first distal joint element 120, and the second distal joint element 122 comprises a respective cover 126, 128, 130, 132.

The covers 126, 128, 130, 132 are shown in detail in **Fig. 19**.

The covers 126, 128, 130, 132 can be made of an electrically insulating material.

In this case, it is desirable that the distance between the metal enclosure of the first intermediate joint element 112 and the second distal joint element 122 and the outer layer of the respective covers 126, 132 is set as 2 mm, thereby ensuring double protection.

In the first distal joint element 120, a distance of 1.6 mm between the metal enclosure of said element 120 and the respective cover 130 is sufficient to ensure double protection, since the electronics for the first distal joint element 120 receive a reduced voltage of 12V.

Additionally or alternatively, the covers 126, 128, 130, 132 can be made of a thermally insulating material.

This facilitates compliance with temperature requirements (e.g., as set forth by IEC/UL60601-1 standard).

The exterior temperature of the robot arm 100 shall be limited in order to prevent the risk of injuries for the patient.

An air gap is formed between each cover 126, 128, 130, 132 and the respective joint element 112, 114, 120, 122, thereby preventing the occurrence of undesired local hotspots.

Preferably, the covers 126, 128, 130, 132 are made of a material which is both electrically and thermally insulating.

As mentioned, the button 148 can be arranged on the cover 130 of the first distal joint element 120 (thus, in the proximity of the end-effector), as shown in **Fig. 16**.

The cover 126 of the first intermediate joint element 112 and the cover 132 of the second distal joint element 122 are removable for cleaning purposes.

The remaining covers 128, 130 are not removable.

In the present embodiment, the ratio between the first to third arm element lengths L1, L2, L3 is:

$$(1.00 +- 10\%) : \{ (1.00 \text{ to } 1.05) +- 10\% \} : (0.60 +- 10\%).$$

Preferably, the ratio between the first to third arm element lengths L1, L2, L3 is:

$$(1.00 +- 3\% \text{ to } +- 5\%) : \{(1.00 \text{ to } 1.05) +- 3 \text{ to } +- 5\%\} : (0.60 +- 3\% \text{ to } +- 5\%);$$

Even more preferably, the first to third arm lengths L1, L2, L3 are set as follows:

first arm element length L1: between 225 and 275 mm, preferably 250 mm;

second arm element length L2: between 231.75 and 283.25 mm, preferably 257.5 mm,

third arm element length L3: between 135 and 165 mm, preferably 150 mm.

The aforementioned ratios have been found by experiment and experience and have proven effective.
In particular, said ratios allow the operator to operate with enhanced dexterity.
Further, the operator is provided with a better vision on the surgical site, free from significant obstructions.
Still further, the risk of collisions between the robot arm 100 and other objects (or even the patient) during manipulation can be largely prevented.
Also, the overall dimensions of the robot arm 100 can be reduced, such that less mass needs to be moved during manipulation.
In the present embodiment, the proximal joint 104 is characterized by the following ranges of motion:

first roll movement: -41° to +41° with respect to the first roll rotation axis, and/or

first pitch movement: -23° to +44° with respect to the first pitch rotation axis.

Additionally or alternatively, the intermediate joint 110 is characterized by the following ranges of motion:

second roll movement: -180° to 180° with respect to the second roll rotation axis, and/or

second pitch movement: -8° to +90° with respect to the second pitch rotation axis,

Additionally or alternatively, the distal joint 118 is characterized by the following ranges of motion:

third roll movement: +-inf, and/or

third pitch movement: -185° to 185° with respect to the third pitch rotation axis.

The design of the bearings 160 plays a significant role in preventing the occurrence of backlash.

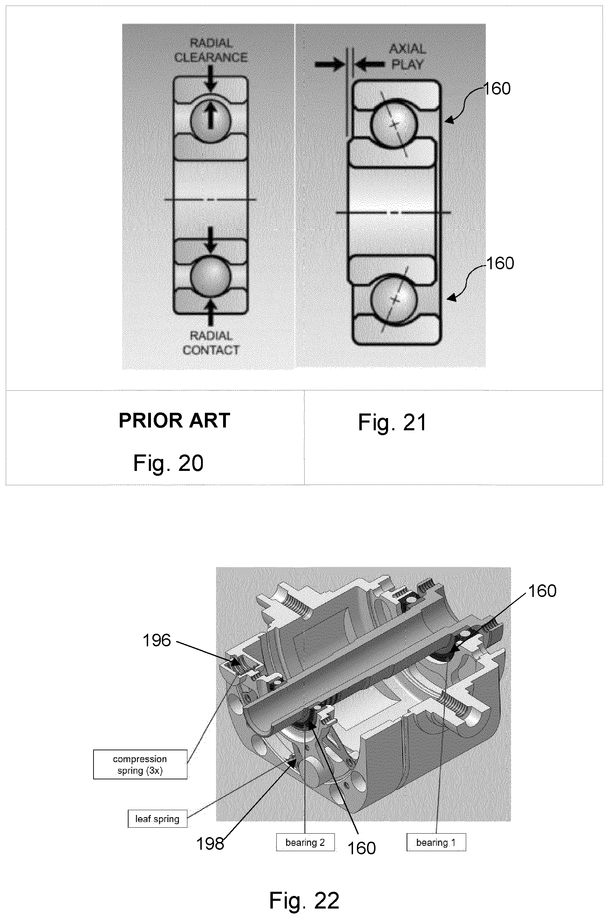

**Fig. 20** shows a known design for a ball bearing system, in particular including a first ball bearing and a second ball bearing.
Here, a clearance exists between the balls in the bearing and the rotor/stator.
This allows ensuring a reduced friction during motion.
However, this also introduces backlash both in the radial and axial direction.
**Fig. 21** shows a ball bearing system as used in the present embodiment.
Similar as above, the ball bearing system includes a first ball bearing 160 and a second ball bearing 160.
Here, the rotor is pre-loaded with respect to the stator, so that no clearance exists between the balls of the bearing and the rotor/stator.
This largely prevents the occurrence of backlash.
Further details of the ball bearing system are shown in **Fig. 22**.
There are two causes of backlash that need being eliminated, namely backlash deriving from tolerances in the shaft of the housing and the second bearing 160, and the play within each bearing 160.
The first cause of backlash can be counteracted by choosing a press fit construction for all components of the ball bearing system, keeping the ball bearings 160 in place.
On the other hand, the second cause of backlash can be counteracted by axially pre-loading the ball bearings 160.
In particular, in the showing configuration, this is achieved by using compression springs 194 (**Fig. 22**).
Also, a leaf spring 196 can be provided to fix the second bearing 160 in the radial direction, at the same time allowing movement in the axial direction, in particular to facilitate pre-loading alignment.

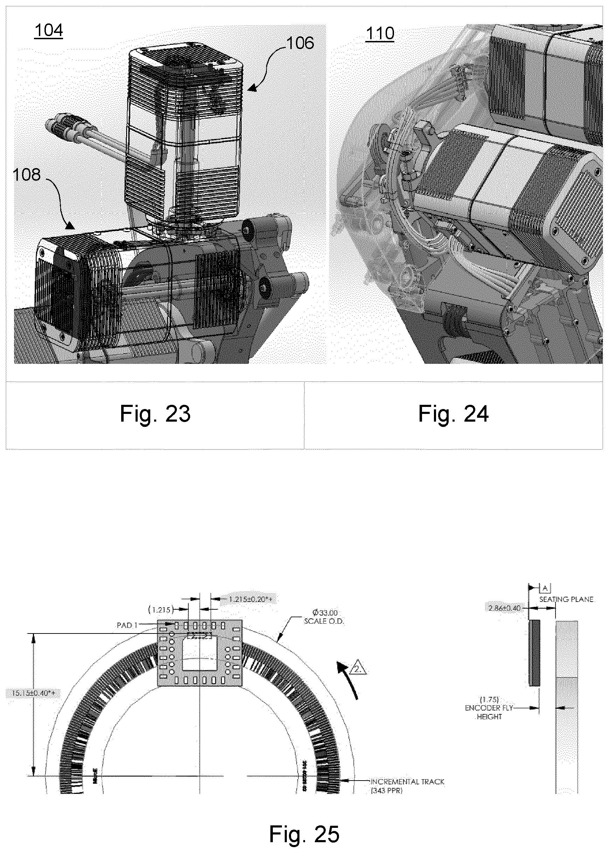

**Figs. 23** schematically shows cable routing in the proximal joint 104.
Here, cable routing is implemented by guiding cables through the inner shaft of the motor stack 152 for both the first proximal joint element 106 and second proximal joint element 108.

This results in the cables being twisted during motion within the motor stack 152.

In order to restrict the motion of the cables purely to the inside of the shaft, the cables are clamped by strain relief clamps provided at both ends of the shaft.

Where the strains relief clamps are clamped on the cable, the sheath of the cable is removed, such that the clamps are directly connected to the shielding of the cable.

This results in the motor stack 152 being grounded.

Clamping must be done at both the rotating and static parts, as they are separated by a non-conductive oil film in the bearing.

**Fig. 24** schematically shows cable routing through the intermediate joint 110.

Here, cable routing around the axis of the second intermediate joint element 114 is carried out around the frame.

At the cable inlet to the motor stack 152, relief clamps are again used, clamping at the shielding of the cable.

On the other hand, for the first intermediate joint element 112, it is not possible routing the cables to the shaft of the motor stack 152 since the range of motion of this element is too large for the torsion capabilities of the cables.

Otherwise stated, routing the cables through the shaft of the motor stack 152 would result in very large cable strains, further generating a large reaction torque at the direct drive 134.

For this reason, a slip ring is provided, where shielding is also achieved through said slip ring.

Accordingly, no clamping is needed at the cable for grounding.

In order to guarantee accuracy of the encoder 156, the alignment of the encoder scale with respect to the encoder read head needs to be done according to specifications, as illustrated in **Fig. 25**.

In particular, these tolerances assume mounting without eccentricity.

Experiments revealed that an eccentricity specification of 0.02 mm results in guaranteed encoder accuracy.

## Mechanical tolerances

Mechanical tolerances are expected in the production of the robot arm 100 according to the invention.

These tolerances may have an impact on the global accuracy of the kinematic model.

The maximum and statistical errors at the tip of the robot arm 100 due to assembly tolerances are illustrated in **Table 1.**

**Table 1** considers tolerances at the tip in different directions, namely x, y, z, Rx, Ry and Rz, in particular resulting from manufacturing tolerances of the individual components of the robot arm 100.

These tolerances are expected to influence the global static accuracy of the robot arm 100.

In particular, these tolerances may introduce a mismatch between the physical robot arm 100 and the geometrical model used to control the robot arm 100.

However, since the effect of these tolerances only affects static performance of the robot arm 100, further considering that the human in the loop will be able to compensate for this, the severity of this issue is expected to be small or even negligible.

A relatively small angular tolerance, e.g. in the second proximal joint element 108, in combination with a relatively large arm (i.e. having an arm length in the range of 250+257.5+150 mm) ensures a large linear tolerance at the tip.

## Robot arm mass and stiffness

Preferably, the robot arm 100 shall be characterized by a stiff but light design.

In particular, a low mass helps increasing the efficiency of the robot arm 100 and also enhances the potential performance by control.

The desirable masses for each of the main components of the robot arm 100 is illustrated in **Table 2.**

**Table 3** provides a list of the heaviest components of the robot arm 100.

In particular, the list refers to brakes and motors, making up 31% of the overall mass of the robotic arm 100.

The closer the mass is located towards the gripper, the more it contributes to the required torque and temperature in the robot arm 100.

This makes especially the brake in first intermediate joint element 112 a large contributor.

For the cart, however, the total mass is even more important.

Here, the largest contributors are the motors in the second proximal joint element 108 and the second intermediate joint element 114.

**Table 1: Error at tip due to assembly tolerances**

|  | x [mm] | y [mm] | z [mm] |
|---|---|---|---|
| **Linear Tolerance [mm]** | 3,73 | 6,53 | 2,78 |
| **Statistical Tolerance [mm]** | 1,55 | 1,91 | 1,24 |

## Table 2: Desirable masses for the main components of the robot arm

| Unit | Mass [kg] |
|---|---|
| **Proximal joint** | **3.96** |
| - First proximal joint element | 1.68 |
| - Second proximal joint element | 2,23 |
| - Proximal joint parts | 0,05 |
| **Intermediate joint** | **5,28** |
| -Intermediate joint parts | 1,12 |
| - First intermediate joint element mount | 1,92 |
| - First intermediate joint element | 1,22 |
| - Second intermediate joint element | 2,24 |
| **Distal joint** | **1,33** |
| - First distal joint element (incl. covers) | 0,23 |
| - Second distal joint element (excl. covers) | 0,92 |
| -Gripper/instr/adapter | 0,175 |
| **PSU-arm parts** | **0,49** |
| **Covering** | **0,25** |
| **Total** | **11.31** |

**Table 3: List of the heaviest components in the robot arm**

| Unit | Rotor mass [g] | Stator mass [g] | Total mass [g] |
|---|---|---|---|
| **Motor for the first proximal joint element** | 80 | 361 | 441 |
| **Motor for the second proximal joint element** | 160 | 717 | 877 |
| **Motor for the first intermediate joint element** | 27 | 149 | 176 |
| **Motor for the second intermediate joint element** | 160 | 717 | 877 |
| **Brakes for the first and second proximal joint elements and the first and second intermediate joint elements** |  |  | 1200(4x300) |
| **Total** | | 3571 | |

**Reference list**

| | |
|---|---|
| 100 | Robot arm |
| 102 | Proximal arm element |
| 104 | Proximal joint |
| 106 | First proximal joint element |
| 108 | Second proximal joint element |
| 110 | Intermediate joint |
| 112 | First intermediate joint element |
| 114 | Second intermediate joint element |

| | |
|---|---|
| 116 | Intermediate arm element |
| 118 | Distal joint |
| 120 | First distal joint element |
| 122 | Second distal joint element |
| 124 | Distal arm element |
| 126 | Cover (of the second pitch) |
| 128 | Cover (of the second roll) |
| 130 | Cover (of the third pitch) |
| 132 | Cover (of the third roll) |
| 134 | Direct drive (motor) |
| 136 | Servo motor |
| 138 | Motor brake |
| 140 | Backlash-free gearbox |
| 142 | Mass compensation means (first spring system) |
| 144 | Mass compensation means (second spring system) |
| 146 | Mass compensation means (third spring system) |
| 148 | Button |
| 150 | End-effector connection means |
| 152 | Motor stack |
| 154 | Housing (motor stack) |
| 156 | Encoder |
| 158 | Drive PCB |
| 160 | Bearing |
| 162 | Heatsink |
| 164 | Intermediate joint PCB |
| 166 | Encoder (second intermediate joint element) |
| 168 | Drive pulley (second intermediate joint element) |
| 170 | Transmission belt (second intermediate joint element) |
| 172 | Steel wire (second intermediate joint element) |
| 174 | Additional pulley (second intermediate joint element) |
| 176 | Load pulley (second intermediate joint element) |
| 178 | Encoder (first distal joint element) |
| 180 | Homing sensor |
| 182 | Button bridge PCB (first distal joint element) |
| 184 | Drive board (first distal joint element) |
| 186 | Balance mass (second distal joint element) |
| 188 | Belt (second distal joint element) |
| 190 | Slip ring (second distal joint element) |
| 194 | Compression spring |
| 196 | Leaf spring |
| | |
| 200 | Surgical robot system |
| 202 | Base column |
| 204 | Suspension arm |
| 206 | Fork element |
| | |
| 300 | Surgical instrument |
| | |
| L1 | First arm element length |
| L2 | Second arm element length |
| L3 | Third arm element length |

**Claims**

1. A robot arm (100) for use in surgery, microsurgery or super-microsurgery, said robot arm (100) comprising:

   at least one arm element (102, 116, 124), and
   at least one direct drive (134),

wherein the at least one direct drive is configured and arranged to actuate the at least one arm element (102, 116, 124).

2. The robot arm (100) according to claim 1, comprising:

a first, proximal arm element (102), said proximal arm element (102) being connected to a first, proximal joint (104) having a first proximal joint element (106) configured to perform a first roll movement and a second proximal joint element (108) configured to perform a first pitch movement, and a second, intermediate joint (110) having a first intermediate joint element (112) configured to perform a second roll movement and a second intermediate joint element (114) configured to perform a second pitch movement, and

a second, intermediate arm element (116), said intermediate arm element (116) being connected to the intermediate joint (110) and a third, distal joint (118) having a first distal joint element (120) configured to perform a third roll movement and a second distal joint element (122) configured to perform a third pitch movement, optionally wherein the robot arm (100) further comprises a third, distal arm element (124), said distal arm element (124) being connected to the distal joint (118),

wherein the robot arm (100) is configured to have six degrees of freedom at its tip, and
wherein the robot arm (100) is configured such that at least one degree of freedom is directly actuated by a respective direct drive (134).

3. The robot arm (100) according to claim 2,

**characterized in that**
each of the first proximal joint element (106), the second proximal joint element (108), the first intermediate joint element (112), the second intermediate joint element (114), and the second distal joint element (122) comprises a direct drive (134) and a motor brake (138), and
the first distal joint element (120) comprises a servo motor (136) with integrated backlash-free gearbox (140).

4. The robot arm (100) according to any one of the preceding claims, **characterized in that**

the robot arm (100) further comprises:

a control means configured to control actuation of the at least one direct drive (134) and/or the servo motor (136), based on preset control parameters, and
at least one sensor, configured to sense disturbances at the tip of a surgical instrument (300), said surgical instrument (300) being connected to the robot arm (100), and provide a feedback signal on the sensed disturbances to said control means, and

wherein the control means is further configured to perform real-time adaption of said control parameters based on a received feedback from the at least one sensor.

5. The robot arm (100) according to any one of the preceding claims, **characterized in that**

the robot arm (100) further comprises a mass compensation means (142, 144, 146),
preferably wherein said mass compensation means (142, 144, 146) is arranged in the second proximal joint element (108) and/or the second intermediate joint element (114).

6. The robot arm (100) according to claim 5,
**characterized in that**

the mass compensation means (142, 144) is arranged in the second proximal joint element (108) and comprises:

a first spring system (142) acting in a first direction, and
a second spring system (144) acting in a second direction, opposite to said first direction,

preferably wherein the first spring system (142) comprises a double spring and the second spring system (144) comprises a single spring.

7. The robot arm (100) according to claim 5 or 6,

**characterized in that**

the mass compensation means (146) is arranged in the second intermediate joint element (114) and comprises a third spring system (146), said third spring system comprising a single spring.

8. The robot arm (100) according to any one of the preceding claims,
**characterized in that**
said six degrees of freedom comprise three translational degrees of freedom and three rotational degrees of freedom.

9. The robot arm (100) according to any one of claims 2 to 8,
**characterized in that**

the ratio between the first to third arm element lengths (L1, L2, L3) is:

$$(1.00 +- 10\%) : \{ (1.00 \text{ to } 1.05) +- 10\% \} : (0.60 +- 10\%);$$

preferably wherein the ratio between the first to third arm element lengths (L1, L2, L3) is:

$$(1.00 +- 3\% \text{ to } +- 5\%) : \{(1.00 \text{ to } 1.05) +- 3 \text{ to } +- 5\% \} : (0.60 +- 3\% \text{ to } +- 5\%);$$

preferably wherein:

the first arm element length (L1) is between 225 and 275 mm, preferably 250 mm;
the second arm element length (L2) is between 231.75 and 283.25 mm, preferably 257.5 mm, and
the third arm element length (L3) is between 135 and 165 mm, preferably 150 mm.

10. The robot arm (100) according to any one of claims 2 to 9, **characterized in that**

the proximal joint (104) is **characterized by** the following ranges of motion:

first roll movement: -41° to +41° with respect to the first roll rotation axis, and/or
first pitch movement: -23° to +44° with respect to the first pitch rotation axis,

and/or
the intermediate joint (110) is **characterized by** the following ranges of motion:

second roll movement: -180° to 180° with respect to the second roll rotation axis, and/or
second pitch movement: -8° to +90° with respect to the second pitch rotation axis,

and/or
the distal joint (118) is **characterized by** the following ranges of motion:

third roll movement: +-inf, and/or
third pitch movement: -185° to 185° with respect to the third pitch rotation axis.

11. The robot arm (100) according to any one of claims 3 to 10, **characterized in that**
the robot arm (100) further comprises a button (148) for simultaneous disengagement of the motor brakes (138).

12. The robot arm (100) according to claim 11,
**characterized in that**

said button (148) is arranged at the tip of the robot arm (100),
preferably wherein said button (148) is arranged on top of the first distal joint element (120) of the robot arm (100).

13. The robot arm (100) according to any one of claims 2 to 12,
**characterized in that**
the first distal joint element (120) comprises an end-effector connection means (150) comprising electrical and mechanical interfaces for connection to an end-effector of the robot arm (100).

**14.** The robot arm (100) according to any one of claims 2 to 13,
**characterized in that**

each of the first proximal joint element (106), the second proximal joint element (108), the first intermediate joint element (112), the second intermediate joint element (114), and the second distal joint element (122) comprises a motor stack (152) having a housing (154) enclosing the direct drive (134) and the motor brake (138), preferably wherein said motor stack (152) further comprises one or more of the following components, arranged within said housing (154):

an encoder (156);
a drive PCB (158), and/or
one or more bearings (160).

**15.** The robot arm (100) according to any one of claims 2 to 14,
**characterized in that**
each of the first intermediate joint element (112), the second intermediate joint element (114), the first distal joint element (120), and the second distal joint element (122) comprises a respective cover (126, 128, 130, 132) made of an electrically and/or thermally insulating material.

**16.** The robot arm (100) according to claim 15,
**characterized in that**
the cover (126) of the first intermediate joint element (112) and the cover (132) of the second distal joint element (122) are removable.

**17.** The robot arm (100) according to any one of claims 2 to 16,
**characterized in that**

the first intermediate joint element (112) and the second distal joint element (122) are configured to receive a voltage of 24V, and
the first distal joint element (120) is configured to receive a voltage of 12V, and
the remaining parts of the robot arm (100) are configured to receive a voltage of 48V.

**18.** A surgical, microsurgical or super-microsurgical robotic system (200), in particular for performing anastomoses, comprising:

a base station;
a base column (202);
a suspension arm (204), carried by the base column (202), the suspension arm (204) being able to rotate about the longitudinal axis of the base column (202);
a fork element (206), carried by the suspension arm (204), and
at least one robot arm (100) according to any one of claims 1 to 17, carried by the fork element (206), the at least one robot arm (100) being configured to carry a respective surgical instrument (300).

| Cases | Vessel diameter (Number of stitches) | Required Precision with human in the loop ±2σ | Vibration Specification Floor | Precision Error Robot (arm on cart) ±2σ |
|---|---|---|---|---|
| Case 3 - Super Microsurgery | 0.3mm(3-4) – 0.5mm(4-5) | 24 μm | Class C | 20 μm |
| Case 2 - Super Microsurgery | 0.5mm(4-5) – 1mm(5-6) | 32 μm | Class A | 30 μm |
| Case 1 - Microsurgery | 1mm(5-6) – 2mm(8-9) | 52 μm | ISO-Operating Theatre | 46 μm |

Fig. 1

Fig. 2

Fig. 3

Fig. 4

Fig. 5

Fig. 6

Fig. 7

Fig. 8

112

Fig. 9

114

Fig. 10

B pitch
motor stack | PCB | B pitch load
encoder

110

152

164

152

114

166

Fig. 11

B roll motor
stack | Mass compensation
system

110

168

170

146

152

114

Fig. 12

Fig. 13

Fig. 14

Fig. 15

Fig. 16

Fig. 17

122

186

158

160

138

160

152

190

188

Balance mass

Drive board

Encoder scale

Brake

Motor stack

Bearing

Belt

Slip ring

Fig. 18

128

132

130

126

Fig. 19

| PRIOR ART Fig. 20 | Fig. 21 |

Fig. 22

104

106

108

Fig. 23

110

Fig. 24

Fig. 25

Europäisches
Patentamt

European
Patent Office

Office européen
des brevets

## EUROPEAN SEARCH REPORT

**Application Number**

EP 24 22 2680

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | EP 4 265 216 A1 (MICROSURE B V [NL])<br>25 October 2023 (2023-10-25)<br>* paragraphs [0038], [0040], [0094], [0097], [0116]; claims 1-15; figures 1,2 * | 1-3,5, 8-18<br><br>4,6,7 | INV.<br>A61B34/30 |
| X | US 2009/163929 A1 (YEUNG BENNY HON BUN [CA] ET AL) 25 June 2009 (2009-06-25)<br>* figures 16a,21i * | 1 | |
| X | US 2004/111183 A1 (SUTHERLAND GARNETTE ROY [CA] ET AL) 10 June 2004 (2004-06-10)<br>* figure 5 * | 1 | |
| Y | EP 3 684 280 B1 (INTUITIVE SURGICAL OPERATIONS [US])<br>10 August 2022 (2022-08-10)<br>* claim 1 * | 4 | |
| Y | US 2021/059783 A1 (HARAGUCHI DAISUKE [JP] ET AL) 4 March 2021 (2021-03-04)<br>* figure 1 * | 6,7 | **TECHNICAL FIELDS SEARCHED (IPC)**<br><br>A61B |

The present search report has been drawn up for all claims

| Place of search | Date of completion of the search | Examiner |
|---|---|---|
| The Hague | 24 July 2025 | Seon, Jean-Antoine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

**Application Number**

**EP 24 22 2680**

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

## LACK OF UNITY OF INVENTION
## SHEET B

**Application Number**

EP 24 22 2680

The Search Division considers that the present European patent application does not comply with the requirements of unity of invention and relates to several inventions or groups of inventions, namely:

    **1. claims: 1-3, 8-18**

        claims 1-3,8-18 relate to the mechanical aspect of a robotic arm

            ---

    **2. claims: 4-7**

        claims 4-7 relates to the control aspect of a robotic arm and a mass compensation system.

            ---

## ANNEX TO THE EUROPEAN SEARCH REPORT
## ON EUROPEAN PATENT APPLICATION NO.

EP 24 22 2680

This annex lists the patent family members relating to the patent documents cited in the above-mentioned European search report.
The members are as contained in the European Patent Office EDP file on
The European Patent Office is in no way liable for these particulars which are merely given for the purpose of information.

24-07-2025

| Patent document cited in search report | | Publication date | Patent family member(s) | | Publication date |
|---|---|---|---|---|---|
| EP 4265216 | A1 | 25-10-2023 | CN | 119317406 A | 14-01-2025 |
| | | | EP | 4265216 A1 | 25-10-2023 |
| | | | EP | 4265219 A1 | 25-10-2023 |
| | | | JP | 2025513505 A | 24-04-2025 |
| | | | KR | 20250003510 A | 07-01-2025 |
| | | | WO | 2023202960 A1 | 26-10-2023 |
| US 2009163929 | A1 | 25-06-2009 | US | 2009163929 A1 | 25-06-2009 |
| | | | US | 2014018821 A1 | 16-01-2014 |
| US 2004111183 | A1 | 10-06-2004 | AU | 2003257309 A1 | 25-02-2004 |
| | | | CA | 2437286 A1 | 13-02-2004 |
| | | | CA | 2633137 A1 | 13-02-2004 |
| | | | EP | 1531749 A2 | 25-05-2005 |
| | | | EP | 2070487 A2 | 17-06-2009 |
| | | | US | 2004111183 A1 | 10-06-2004 |
| | | | US | 2007032906 A1 | 08-02-2007 |
| | | | US | 2008004632 A1 | 03-01-2008 |
| | | | US | 2008161677 A1 | 03-07-2008 |
| | | | US | 2008161830 A1 | 03-07-2008 |
| | | | US | 2010063630 A1 | 11-03-2010 |
| | | | WO | 2004014244 A2 | 19-02-2004 |
| EP 3684280 | B1 | 10-08-2022 | CN | 111315309 A | 19-06-2020 |
| | | | CN | 116370080 A | 04-07-2023 |
| | | | EP | 3684280 A2 | 29-07-2020 |
| | | | EP | 4140431 A1 | 01-03-2023 |
| | | | JP | 6880324 B2 | 02-06-2021 |
| | | | JP | 2021502173 A | 28-01-2021 |
| | | | KR | 20200052980 A | 15-05-2020 |
| | | | KR | 20210024229 A | 04-03-2021 |
| | | | US | 2020261169 A1 | 20-08-2020 |
| | | | US | 2021177535 A1 | 17-06-2021 |
| | | | US | 2023248454 A1 | 10-08-2023 |
| | | | US | 2025025247 A1 | 23-01-2025 |
| | | | WO | 2019094794 A2 | 16-05-2019 |
| US 2021059783 | A1 | 04-03-2021 | CN | 111936027 A | 13-11-2020 |
| | | | EP | 3777641 A1 | 17-02-2021 |
| | | | JP | 6751943 B2 | 09-09-2020 |
| | | | JP | 2018175863 A | 15-11-2018 |
| | | | US | 2021059783 A1 | 04-03-2021 |
| | | | WO | 2019193775 A1 | 10-10-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2731535 A
- EP 22169326 A